# EUROPEAN PATENT APPLICATION

(11) **EP 4 640 136 A1**
(43) Date of publication of application: **29.10.2025**
(21) Application number: 23905341.6
(22) Date of filing: 15.09.2023
(51) Int. Cl.: A61B 5/00

(54) **DENTAL IMAGING APPARATUS**

(30) Priority: 23.12.2022 CN 202211667767
(71) Applicant: Shining 3D Tech Co., Ltd., Hangzhou, Zhejiang 311258 (CN)
(72) Inventor: MA, Chao, Hangzhou, Zhejiang 311258 (CN); ZHAO, Xiaobo, Hangzhou, Zhejiang 311258 (CN); CHEN, Xiaojun, Hangzhou, Zhejiang 311258 (CN)
(74) Representative: Metida
(86) International application number: PCT/CN2023/119083
(87) International publication number: WO 2024/131165

(57) **Abstract**

The present disclosure relates to a technical field of dental equipment, and in particular to a dental photography device. The dental photography device comprises a mouth prop and an image capture device (3), and the image capture device (3) is oriented towards an opening area of the mouth prop, and the mouth prop and the image capture device (3) are connected by an adjustment mechanism, and a relative position between the mouth prop and the image capture device (3) is adjustable by the adjustment mechanism; the adjustment mechanism comprises a translation mechanism that enables a relative translation between the mouth prop and the image capture device (3), and/or the adjustment mechanism comprises a rotation mechanism that enables a relative rotation between the mouth prop and the image capture device (3). By incorporating the mouth prop, the image capture device (3), and the adjustment mechanism, the relative position between the mouth prop and the image capture device (3) can be adjusted through the adjustment mechanism, and then dental information in patient's oral cavity is captured by the image capture device (3), enabling the patient to easily perform imaging at home, which meets the needs of real-time orthodontic monitoring and improves user experience.

## Description

### FIELD

The present disclosure relates to a technical field of dental equipment, and in particular to a dental photography device.

### BACKGROUND

Teeth not only have an important chewing function for humans, but also have an aesthetic effect. Therefore, with the improvement of people's living standards, the demand for orthodontics treatment is on the rise, especially for teenagers. Orthodontic treatment usually corrects patients' uneven teeth, malocclusion, facial contour problems and other problems, and rearranges and positions teeth to achieve the desired effect.

At present, conventional orthodontic testing mainly relies on regular communication or supervision between doctors and patients, and relies on patients to go to the clinic regularly for examination and correction. Once a doctor finds that the orthodontic effect is not growing in a direction of a plan, timely adjustments are made to ensure the smooth progress of the treatment. Frequent visits to the doctor make the patient experience poor, therefore, an equipment capable of photographing teeth by itself is urgently needed to solve the above problem.

### SUMMARY

### (1) Technical Issues To Be Resolved.

At present, conventional orthodontic testing mainly relies on regular communication or supervision between doctors and patients, and relies on patients to go to the clinic regularly for examination and correction. Once a doctor finds that the orthodontic effect is not growing in a direction of a plan, timely adjustments are made to ensure the smooth progress of the treatment. Frequent visits to the doctor make the patient experience poor, therefore, an equipment capable of photographing teeth by itself is urgently needed to solve the above problem.

### (2) Technical Solution

According to various embodiments disclosed in the present disclosure, a dental photography device is provided.

A dental photography device comprises a mouth prop and an image capture device, and the image capture device is oriented towards an opening area of the mouth prop, the mouth prop and the image capture device are connected by an adjustment mechanism, and a relative position between the mouth prop and the image capture device is adjustable by the adjustment mechanism;
the adjustment mechanism comprises a translation mechanism that enables a relative translation between the mouth prop and the image capture device, and/or the adjustment mechanism comprises a rotation mechanism that enables a relative rotation between the mouth prop and the image capture device.

As an optional embodiment of the present disclosure, the mouth prop comprises a fixing base and a plurality of arc-shaped expansion blades, and the fixing base comprises a support ring and a mounting frame, the arc-shaped expansion blades are movably mounted on an inner side of the support ring through the mounting frame, and are movable in a direction toward or away from a center of the support ring, and the plurality of arc-shaped expansion blades define the opening area.

As an optional embodiment of the present disclosure, the mounting frame comprises a mounting bracket, a connecting block and a connecting plate, and the support ring is threaded through and connected to the mounting bracket, and the connecting block is located on an inner side of the support ring and connected to the arc-shaped expansion blades, the connecting plate is movably arranged on the mounting bracket and connected to the connecting block, and the connecting plate is capable of driving the connecting block to move in a direction toward or away from the center of the support ring.

As an optional embodiment of the present disclosure, the mounting frame further comprises a limiting component, and the limiting component is disposed between the mounting bracket and the connecting plate for restricting movement of the connecting plate.

As an optional embodiment of the present disclosure, the mounting bracket comprises a bottom housing and a top cover, and the bottom housing is connected to the top cover, and the support ring is connected to the bottom housing and positioned between the bottom housing and the top cover, and the connecting plate is inserted into the top cover, and the top cover is equipped with an inserting slot, and the connecting plate is inserted into the inserting slot and is capable of moving along the inserting direction.

As an optional embodiment of the present disclosure, the mounting bracket comprises a bottom housing and a top cover, and the bottom housing is connected to the top cover, and the support ring is connected to the bottom housing and positioned between the bottom housing and the top cover, and the bottom housing and/or the top cover is equipped with a through hole for the connecting plate to pass through.

As an optional embodiment of the present disclosure, the mounting bracket further comprises a side plate, and the side plate is located on an outer side of the support ring, and the connecting plate is connected to the side plate, and the side plate is capable of sealing an opening of a cavity formed by the bottom housing and the top cover, and is embedded within the opening.

As an optional embodiment of the present disclosure, the translation mechanism comprises a base, and the mouth prop and/or the image capture device is slidably arranged on the base along a first direction.

As an optional embodiment of the present disclosure, the mouth prop is equipped with a connecting portion, and the connecting portion is slidably arranged on the base, one of the connecting portion and the base is equipped with a first slide groove extending along the first direction, and the other is equipped with a first sliding block, the first sliding block is positioned within the first slide groove and slidably engages with the first slide groove.

As an optional embodiment of the present disclosure, the rotating mechanism comprises a mounting base, and the image capture device is mounted on the mounting base and is capable of rotating around an axis extending along a second direction, and the second direction is perpendicular to the first direction.

As an optional embodiment of the present disclosure, the mounting base is slidably arranged on the base, one of the mounting base and the base is equipped with a second slide groove extending along the first direction, and the other is equipped with a second sliding block, and the second sliding block is positioned within the second slide groove and slidably engages with the second slide groove.

As an optional embodiment of the present disclosure, the rotating mechanism further comprises a rotating shaft extending along the second direction, and the rotating shaft is rotatably disposed on the mounting base and connected to the image capture device.

Other features and advantages of the present disclosure will be elaborated in the subsequent description, and some will become apparent from the description or be understood through the implementation of the present disclosure. The objectives and other advantages of the present disclosure are achieved and attained through the structures particularly pointed out in the description, claims, and drawings. T he details of one or more embodiments of the present disclosure are presented in the accompanying drawings and the description below.

In order to make the aforementioned objectives, features, and advantages of the present disclosure more comprehensible, optional embodiments are specifically described below in conjunction with the accompanying drawings for detailed explanation.

### BRIEF DESCRIPTION OF THE DRAWINGS

In order to more clearly illustrate embodiments of the present invention or technical solutions in the prior art, the drawings used in the embodiments or the description of the prior art will be briefly introduced below. Obviously, for ordinary technicians in this field, other drawings can be obtained based on these drawings without paying any creative labor.
FIG. 1 is a first schematic diagram of a partial structure of a dental photography device according to an embodiment of the present disclosure;
FIG. 2 is a partial disassembly schematic diagram of the dental photography device according to the embodiment of the present disclosure;
FIG. 3 is a disassembled schematic diagram of a mounting frame according to an embodiment of the present disclosure;
FIG. 4 is another disassembled schematic diagram of the mounting frame according to an embodiment of the present disclosure;
FIG. 5 is a second schematic diagram of a partial structure of the dental photography device according to an embodiment of the present disclosure.

### DETAILED DESCRIPTION

In order to more clearly understand the above-mentioned objectives, features and advantages of the present disclosure, the scheme of the present disclosure will be further described below. It should be noted that the embodiments of the present disclosure and the features in the embodiments can be combined with each other without conflict.

In the following description, many specific details are set forth to facilitate a full understanding of the present disclosure, but the present disclosure may also be implemented in other ways different from those described herein; it is obvious that the embodiments in the specification are only part of the embodiments of the present disclosure, rather than all of the embodiments.

The terms "first" and "second" in the specification and claims of the present disclosure are used to distinguish different objects rather than to describe a specific order of objects. For example, a first camera and a second camera are used to distinguish different cameras rather than to describe a specific order of cameras.

In the embodiments of the present disclosure, words "exemplary" or "for example" are used to indicate examples, illustrations or descriptions. Any embodiment or design described as "exemplary" or "for example" in the embodiments of the present disclosure should not be interpreted as being more preferred or more advantageous than other embodiments or designs. Specifically, the use of words such as "exemplary" or "for example" is intended to present related concepts in a specific way. In addition, in the description of the embodiments of the present disclosure, unless otherwise specified, the meaning of "multiple" refers to two or more.

Existing dust collection component usually need to be disassembled and assembled on a top of a base station, which not only takes up the space on the top of the base station, but also forms a visual blockage for a pipe openings inside the base station, which can easily cause installation deviations of the dust collection component. As a result, the garbage sucked in by the base station cannot be completely collected by the dust collection component, thereby contaminating the body of the base station and contaminating the ground again when the dust collection component is removed.

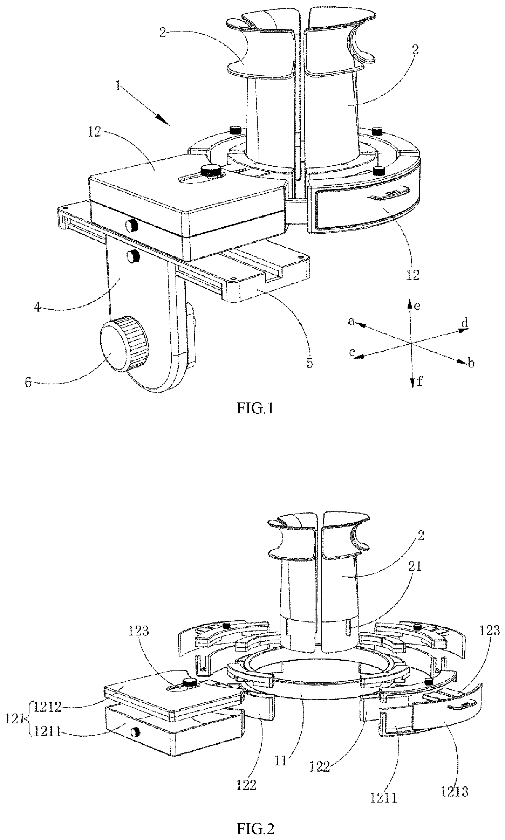

As shown in FIGS. 1 to 5, the embodiment of the present disclosure provides a dental photography device, which is mainly used by patients to take photos or videos of their own teeth for the convenience of doctors' examination. Specifically, the dental photography device includes a mouth prop and an image capture device 3, and the image capture device 3 is oriented towards an opening area of the mouth prop. The mouth prop and the image capture device 3 are connected by an adjustment mechanism, and a relative position between the mouth prop and the image capture device 3 is adjustable through the adjustment mechanism. The adjustment mechanism includes a translation mechanism that enables a relative translation between the mouth prop and the image capture device 3, and/or the adjustment mechanism includes a rotation mechanism that enables a relative rotation between the mouth prop and the image capture device 3.

It can be understood that by incorporating the mouth prop, the image capture device 3 and the adjustment mechanism, the relative positions between the mouth prop and the image capture device 3 can be adjustable through the adjustment mechanism, and then the teeth in an oral cavity can be photographed by the image capture device 3 by the opening area of the mouth prop. Patients can also easily take pictures at home by using the dental photography device, which meets needs of real-time orthodontic detection and improves user experience.

As shown in FIGS. 1 to 3, the above-mentioned mouth prop includes a fixing base 1 and a plurality of arc-shaped expansion blades 2. The fixing base 1 includes a support ring 11 and a mounting frame 12. The arc-shaped expansion blades 2 are movably mounted on an inner side of the support ring 11 by the mounting frame 12 and are movable in a direction toward or away from a center of the support ring 11. The plurality of arc-shaped expansion blades 2 define the opening area.

It can be understood that the opening area of the mouth prop is formed between the plurality of arc-shaped expansion blades 2, and the patient's lips and cheeks are stretched open by moving the arc-shaped expansion blades 2 in a direction away from the center of the support ring 11, which is convenient for the image capture device 3 to take pictures. Moreover, the movement of the arc-shaped expansion blades 2 in a direction toward or away from the center of the support ring 11 can make the dental photography device adapt to the oral cavity of different patients, further improving user experience.

Exemplarily, as shown in FIG. 2, the support ring 11 is a circular ring. Optionally, the support ring 11 may also be a square ring, or a polygonal ring (with more than four sides).

There are plurality of mounting frames 12, which are spaced apart along the circumference of the support ring 11. Each of mounting frames 12 is connected to one arc-shaped expansion blade 2 to drive the arc-shaped expansion blade 2 to move in a direction toward or away from the center of the support ring 11.

Referring to FIG. 2, the mounting frame 12 includes a mounting bracket 121, a connecting block 122 and a connecting plate 123. The support ring 11 is threaded through and connected to the mounting bracket 121. The connecting block 122 is located inside the support ring 11 and connected to the arc-shaped expansion blade 2. The connecting plate 123 is movably arranged on the mounting bracket 121 and connected to the connecting block 122. The connecting plate 123 is capable of driving the connecting block 122 to move in a direction toward or away from the center of the support ring 11. The connecting plate 123 is provided to facilitate the patient's operation, so that the position of the arc-shaped expansion blades 2 can be easily adjusted.

Furthermore, the mounting frame 12 further includes a limiting component 124 disposed between the mounting bracket 121 and the connecting plate 123 to restrict the movement of the connecting plate 123. Specifically, the connecting plate 123 is provided with a plurality of fixing holes disposed along its moving direction, and the limiting component 124 can extend into the fixing holes to limit the movement of the connecting plate 123. The plurality of fixing holes can be connected or spaced apart.

Optionally, the limiting component 124 is threadedly connected to the mounting bracket 121, and the limiting component 124 is screwed to extend into or out of one fixing hole. When the limiting component 124 extends into the fixing hole, the movement of the connecting plate 123 can be restricted. Alternatively, the limiting component 124 is threaded through and set on the mounting bracket 121, and can be threadedly connected to the fixing hole. When the limiting component 124 is threadedly connected to one of the fixing holes, the movement of the connecting plate 123 can also be restricted.

Furthermore, the connection block 122 is equipped with a slot 1221, and the arc-shaped expansion blade 2 is equipped with a plug-in block 21, and the plug-in block 21 can be plugged into the slot 1221 to achieve a connection between the arc-shaped expansion blade 2 and the connection block 122. Such a configuration can avoid the arc-shaped expansion blade 2 being too thick due to the slot being provided on the arc-shaped expansion blade 2, and also facilitates the arc-shaped expansion blade 2 to have a certain elasticity, thereby preventing the arc-shaped expansion blade 2 from damaging the patient's lips and cheeks.

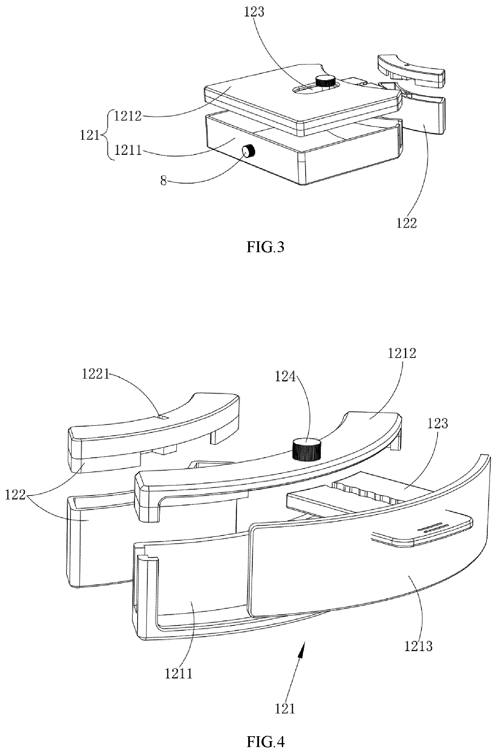

Optionally, referring to FIG. 3, the mounting bracket 121 includes a bottom housing 1211 and a top cover 1212, and the bottom housing 1211 is connected to the top cover 1212, and the support ring 11 is connected to the bottom housing 1211 and positioned between the bottom housing 1211 and the top cover 1212. The connecting plate 123 is inserted into the top cover 1212, and the top cover 1212 is equipped with an insertion slot (not shown in the figure), the connecting plate 123 is inserted into the insertion slot and is movable along the insertion direction. At this time, the limiting component 124 is connected to the connecting plate 123 and is movable synchronously with the connecting plate 123. The top cover 1212 is equipped with a strip hole for the limiting component 124 to pass through, and the strip hole extends along the insertion direction.

Furthermore, referring again to FIG. 3, the bottom housing 1211 and the top cover 1212 of the mounting bracket 121 of the mounting frame 12 surround a substantially quadrilateral cavity, and the support ring 11 passes through the cavity and is connected to the bottom housing 1211. The connecting plate 123 of the mounting frame 12 is located in the cavity.

Optionally, referring to FIG. 4 , the mounting bracket 121 includes the bottom housing 1211 and the top cover 1212, and the bottom housing 1211 is connected to the top cover 1212, and the support ring 11 is connected to the bottom housing 1211 and positioned between the bottom housing 1211 and the top cover 1212, and the bottom housing 1211 and/or the top cover 1212 defines a through hole for the connecting plate 123 to pass through. In this way, the connecting plate 123 can pass through the through hole to connect with the arc-shaped expansion blade 2, so that the connecting plate 123 and the arc-shaped expansion blade 2 are linked without restriction. The limiting component 124 is arranged on the top cover 1212.

It is understandable that the through hole for the connecting plate 123 to pass through may be opened on the bottom housing 1211, or the through hole for the connecting plate 123 to pass through may be opened on the top cover 1212, or the bottom housing 1211 and the top cover 1212 may jointly form the through hole for the connecting plate 123 to pass through. In this case, both the bottom housing 1211 and the top cover 1212 have one half hole. After the bottom housing 1211 and the top cover 1212 are connected, the two half holes form a through hole for the connecting plate 123 to pass through.

Alternatively, referring to FIG. 4, a cavity with an opening on one side is formed between the bottom housing 1211 and the top cover 1212 of the mounting bracket 12 of the mounting frame 121, and the support ring 11 is inserted into the cavity and connected to the bottom housing 1211. Such a configuration can reduce the weight of the mounting bracket 121, thereby reducing the weight of the entire dental photography device.

Furthermore, as shown in FIG. 4, the mounting bracket 121 further includes a side plate 1213, and the side plate 1213 is located on an outer side of the support ring 11, and the connecting plate 123 is connected to the side plate 1213. The side plate 1213 is capable of sealing the opening of the cavity formed by the bottom housing 1211 and the top cover 1212 and is embedded within the opening. When the side plate 1213 moves inward with the connecting plate 123, the bottom housing 1211 and the top cover 1212 (or the support ring 11) restrict the connecting plate 123 to prevent the connecting plate 123 from exceeding the stroke and detaching from the bottom housing 1211 and the top cover 1212. In addition, the side plate 1213 can also provide a counterweight for an end of the connecting plate 123, and the end of the connecting plate 123 is away from the connecting block 122, thereby improving the stability of the first connecting plate 123, and when the patient does not need to take photos, the side plate 1213 is capable of sealing the opening and be embedded in the opening, which is not only beautiful, but also can prevent dust from accumulating in the cavity.

Furthermore, the connecting plate 123 is threaded through the side plate 1213, that is, one end of the connecting plate 123 away from the corresponding arc-shaped expansion blade 2 extends out of the side plate 1213, so that it is convenient to push and pull the connecting plate 123.

Exemplarily, as shown in FIGS. 2 to 4, four mounting frames 12 are provided, wherein the structure of one mounting frame 12 is shown in FIG. 3, and the structures of the other three mounting frames 12 are shown in FIG. 4. Four arc-shaped mouth-expansion blades 2 are provided correspondingly, and the four arc-shaped mouth-expansion blades 2 are used together to open the lips and cheeks of the patient. Optionally, the number of the arc-shaped mouth-expansion blades 2 can also be set to two, three, or more than four. Of course, the structures of the mounting frames 12 can also be the structures of the mounting frames 12 in FIG. 3, or the structures of the mounting frames 12 in FIG. 2.

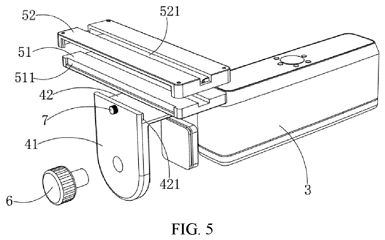

As shown in FIG. 1 and FIG. 5, the translation mechanism comprises a base 5, and the mouth prop and/or the image capture device 3 is slidably arranged on the base 5 along a first direction. The first direction is a "ab" direction shown in FIG. 1.

Furthermore, the rotating mechanism includes a mounting base 4, and the image capture device 3 is mounted on the mounting base 4, and can rotate around an axis extending along a second direction, and the second direction is perpendicular to the first direction. The second direction is a "cd" direction shown in FIG. 1.

It is understandable that, among the mouth prop and the mounting base 4, only the mouth prop can be slidably arranged on the base 5 along the first direction, or only the mounting base 4 can be slidably arranged on the base 5 along the first direction, or both the mouth prop and the mounting base 4 can be slidably arranged on the base 5 along the first direction.

For example, as shown in FIG. 5, the mounting base 4 is slidably arranged on the base 5 along the first direction, and the mouth prop is slidably arranged on the base 5. The first direction, the second direction and a central axis direction of the support ring 11 are perpendicular to each other. The central axis direction of the support ring 11 is the "ef" direction shown in FIG. 1.

By incorporating the mounting base 4 and the base 5, a position of the arc-shaped expansion blade 2 can be adjusted to open the patient's lips and cheeks, and then a position of the fixing base 1 and a position of the image capture device 3 can be adjusted in the first direction, and then a shooting angle of the image capture device 3 can be adjusted during shooting to achieve three-dimensional photography of the patient's teeth in the mouth and improve shooting accuracy.

Furthermore, the mouth prop is equipped with a connecting portion, which is slidably arranged on the base 5. One of the connecting portion and the base 5 is equipped with a first slide groove 521 extending along the first direction, and the other is equipped with a first sliding block (not shown in the figure), which is positioned within the first slide groove 521 and slidably engages with the first slide groove 521. In this way, the mouth prop can slide relative to the base 5 in the first direction, and the first slide groove 521 and the first sliding block are mutually limited, so that a sliding relationship between the mouth prop and the base 5 can be kept stable.

Specifically, as shown in FIG. 1 and FIG. 5, the first sliding block is provided on the connecting portion, and a first slide groove 521 is provided on the base 5. Optionally, the connecting portion is integrally provided with one of the mounting frames 12, that is, the connecting portion and the mounting frame 12 are integrally provided to form the bottom housing 1211, and the bottom housing 1211 is the bottom housing 1211 shown in FIG. 3. Optionally, the connecting portion can also be separately provided with the mounting frame 12, in which case the connecting portion is connected to the mounting frame 12, so that the mounting frame 12 is driven by sliding the connecting portion relative to the base 5, thereby the entire mouth prop slides relative to the base 5 in the first direction.

It should be noted that the relative sliding or stillness between the connecting portion and the base 5 is controlled by the stop switch 8 provided on the bottom housing 1211. The stop switch 8 is capable of tightening the first sliding block, causing the first sliding block to presses against the side wall of the first slide groove 521, thereby keeping the first sliding block stationary within the first sliding groove 521, at this point, the connecting portion remains stationary relative to the base 5. When the stop switch 8 releases the first sliding block, the first sliding block can slide in the first slide groove 521, and the connecting portion can slide relative to the base 5.

Furthermore, one of the mounting base 4 and the base 5 is equipped with a second slide groove 511 extending in the first direction, and the other is equipped with a second sliding block 421, and the second sliding block 421 is positioned within the second slide groove 511 and slidably engages with the second slide groove. In this way, the mounting base 4 can slide relative to the base 5 in the first direction, and the second slide groove 511 and the second sliding block 421 are mutually limited, so that the sliding relationship between the mounting base 4 and the base 5 can be kept stable.

Specifically, as shown in FIG. 5, the second sliding block 421 is disposed on the mounting base 4, and the second sliding groove 511 is disposed on the base 5.

Furthermore, the second slide groove 511 and the second sliding block 421 are both equipped with two, and one of the second slide grooves 511 corresponds one of the second sliding blocks 421. The stability of the relative sliding between the mounting base 4 and the base 5 can be further improved. Specifically, the mounting base 4 is equipped with two second sliding blocks 421, and the two second sliding blocks 421 are spaced apart in the second direction. Correspondingly, the base 5 is equipped with two second slide grooves 511, and the two second slide grooves 511 are also spaced apart in the second direction. Optionally, the mounting base 4 may be equipped with two second slide grooves 511, and the base 5 may be equipped with two second sliding blocks 421, or the mounting base 4 may be equipped with one second slide groove 511 and one second sliding block 421, and the base 5 may be equipped with one second slide groove 511 and one second sliding block 421.

Referring to FIG. 5, the mounting base 4 includes a first portion 41 and a second portion 42 connected to each other, wherein the first portion 41 is a plate-shaped structure, and the second portion 42 is an L-shaped structure. The second portion 42 includes a first plate and a second plate connected to each other, and the first portion 41 is connected to the first plate, and the first portion and the second plate are both equipped with one of the second sliding blocks 421. It should be noted that, in order to reduce the number of parts and components and reduce the assembly process, the first portion 41 and the second portion 42 constituting the mounting base 4 are integrally processed and formed. Optionally, the first portion 41 and the second portion 42 may also be provided separately.

The base 5 includes a first guide rail 51 and a second guide rail 52, and the second guide rail 52 is connected to the first guide rail 51, and the first guide rail 51 is equipped with one second slide groove 511, and the second guide rail 52 is equipped with the first slide groove 521, and the second guide rail 52 is covered on the top of the first portion 41. In this way, the first portion 41 can be covered to improve the aesthetics of the dental photography device.

Furthermore, the second slide groove 511 does not pass through both ends of the first guide rail 51 in the first direction, that is, the second slide groove 511 is not a through groove in the first direction, so that the slider 421 can be limited in the first direction to prevent the second sliding block 421 from escaping from the second slide groove 511.

As shown in FIG. 5, the rotating mechanism further includes a rotating shaft 6 extending along the second direction, and the rotating shaft 6 is rotatably disposed on the mounting base 4 and connected to the image capture device 3. The rotating shaft 6 is disposed through the first portion 41 and the first plate. By providing the rotating shaft 6, an angle of the image capture device 3 can be adjustable through rotating the rotating shaft 6, so that the user can adjust the shooting angle of the image capture device 3.

Furthermore, the rotating shaft 6 passes through the mounting base 4 and is threadedly connected to the mounting base 4, so that the stability of the image capture device 3 can be maintained when the rotating shaft 6 is rotated.

Referring to FIG. 5 again, the dental photography device further includes a fixing component 7, and the fixing component 7 is disposed on the mounting base 4, and is capable of tightening or loosening the base 5.

Specifically, the fixing component 7 is arranged on the first portion 41, and is capable of tightening or loosening the first guide rail 51. Optionally, the fixing component 7 can be arranged to be threadedly connected to the first portion 41, and the fixing component 7 can be screwed to tighten or loosen the first guide rail 51. When the fixing component 7 presses against the first guide rail 51, the relative sliding between the base 5 and the mounting base 4 can be limited. When the fixing component 7 releases the first guide rail 51, the base 5 and the mounting base 4 can slide relative to each other. The structure is simple and easy to operate. The fixing component 7 can also be arranged as a stop switch. When the stop switch is pressed, it can tighten the base 5 to limit the mounting base 4 from sliding relative to the base 5.

The technical features of the above embodiments may be combined arbitrarily. To make the description concise, not all possible combinations of the technical features in the above embodiments are described. However, as long as there is no contradiction in the combination of these technical features, they should be considered to be within the scope of this specification.

The above embodiments only express several implementation methods of the present disclosure, and the descriptions thereof are relatively specific and detailed, but they cannot be understood as limiting the scope of the application. It should be pointed out that, for those of ordinary skill in the art, several variations and improvements can be made without departing from the concept of the present disclosure, and these all belong to the protection scope of the present disclosure. Therefore, the protection scope of the patent of the present disclosure shall be subject to the attached claims.

### Industrial Applicability

The present invention discloses a dental photography device, by incorporating the mouth prop, the image capture device, and the adjustment mechanism, the relative position between the mouth prop and the image capture device can be adjusted through the adjustment mechanism, and then the teeth in the oral cavity can be photographed by the image capture device through the opening area of the mouth prop, enabling the patient to easily perform imaging at home, which meets the needs of real-time orthodontic monitoring and improves user experience.

## Claims

1. A dental photography device, **characterized in that** the dental photography device comprises a mouth prop and an image capture device (3), and the image capture device (3) is oriented towards an opening area of the mouth prop, the mouth prop and the image capture device (3) are connected by an adjustment mechanism, and a relative position between the mouth prop and the image capture device (3) is adjustable by the adjustment mechanism;
the adjustment mechanism comprises a translation mechanism that enables a relative translation between the mouth prop and the image capture device (3), and/or the adjustment mechanism comprises a rotation mechanism that enables a relative rotation between the mouth prop and the image capture device (3).

2. The dental photography device according to claim 1, **characterized in that** the mouth prop comprises a fixing base (1) and a plurality of arc-shaped expansion blades (2), and the fixing base (1) comprises a support ring (11) and a mounting frame (12), the arc-shaped expansion blades (2) are movably mounted on an inner side of the support ring (11) through the mounting frame (12), and are movable in a direction toward or away from a center of the support ring (11), and the plurality of arc-shaped expansion blades (2) define the opening area.

3. The dental photography device according to claim 2, **characterized in that** the mounting frame (12) comprises a mounting bracket (121), a connecting block (122) and a connecting plate (123), and the support ring (11) is threaded through and connected to the mounting bracket (121), and the connecting block (122) is located on an inner side of the support ring (11) and connected to the arc-shaped expansion blades (2), the connecting plate (123) is movably arranged on the mounting bracket (121) and connected to the connecting block (122), and the connecting plate (123) is capable of driving the connecting block (122) to move in a direction toward or away from the center of the support ring (11).

4. The dental photography device according to claim 3, **characterized in that** the mounting frame (12) further comprises a limiting component (124), and the limiting component (124) is disposed between the mounting bracket (121) and the connecting plate (123) for restricting movement of the connecting plate (123).

5. The dental photography device according to claim 4, **characterized in that** the mounting bracket (121) comprises a bottom housing (1211) and a top cover (1212), and the bottom housing (1211) is connected to the top cover (1212), and the support ring (11) is connected to the bottom housing (1211) and positioned between the bottom housing (1211) and the top cover (1212), and the connecting plate (123) is inserted into the top cover(1212), and the top cover (1212) is equipped with an inserting slot, and the connecting plate (123) is inserted into the inserting slot and is capable of moving along the inserting direction.

6. The dental photography device according to claim 3, **characterized in that** the mounting bracket (121) comprises a bottom housing (1211) and a top cover (1212), and the bottom housing (1211) is connected to the top cover (1212), and the support ring (11) is connected to the bottom housing (1211) and positioned between the bottom housing (1211) and the top cover (1212), and the bottom housing (1211) and/or the top cover (1212) is equipped with a through hole for the connecting plate (123) to pass through.

7. The dental photography device according to claim 6, **characterized in that** the mounting bracket (121) further comprises a side plate (1213), and the side plate (1213) is located on an outer side of the support ring (11), and the connecting plate (123) is connected to the side plate (1213), and the side plate (1213) is capable of sealing an opening of a cavity formed by the bottom housing (1211) and the top cover (1212), and is embedded within the opening.

8. The dental photography device according to claim 1, **characterized in that** the translation mechanism comprises a base (5), and the mouth prop and/or the image capture device (3) is slidably arranged on the base (5) along a first direction.

9. The dental photography device according to claim 8, **characterized in that** the mouth prop is equipped with a connecting portion, and the connecting portion is slidably arranged on the base (5), one of the connecting portion and the base (5) is equipped with a first slide groove (521) extending along the first direction, and the other is equipped with a first sliding block, the first sliding block is positioned within the first slide groove (521) and slidably engages with the first slide groove (521).

10. The dental photography device according to claim 8, **characterized in that** the rotating mechanism comprises a mounting base (4), and the image capture device (3) is mounted on the mounting base (4) and is capable of rotating around an axis extending along a second direction, and the second direction is perpendicular to the first direction.

11. The dental photography device according to claim 10, **characterized in that** the mounting base (4) is slidably arranged on the base (5), one of the mounting base (4) and the base (5) is equipped with a second slide groove (511) extending along the first direction, and the other is equipped with a second sliding block (421), and the second sliding block (421) is positioned within the second slide groove (511) and slidably engages with the second slide groove (511).

12. The dental photography device according to claim 10, **characterized in that** the rotating mechanism further comprises a rotating shaft (6) extending along the second direction, and the rotating shaft (6) is rotatably disposed on the mounting base (4) and connected to the image capture device (3).
